# EUROPÄISCHE PATENTSCHRIFT

(11) **EP 1 053 998 B1**
(45) Veröffentlichungstag und Bekanntmachung des Hinweises auf die Patenterteilung: **09.04.2003**
(21) Anmeldenummer: 00109114.9
(22) Anmeldetag: 04.05.2000
(51) Int. Cl.: C07C 263/20

(54) **Verfahren zur Reinigung von organischen Isocyanaten**
Process for the purification of organic isocyanates
Procédé pour la purification d'isocyanates organiques

(30) Priorität: 17.05.1999 DE 19922572
(43) Veröffentlichungstag der Anmeldung: 22.11.2000
(73) Patentinhaber: BAYER AG, 51368 Leverkusen (DE)
(72) Erfinder: Danielmeier, Karsten, Dr., Bethel Park, PA 15102 (US); Mager, Dieter, Dr., 51373 Leverkusen (DE); Halpaap, Reinhard, Dr., 51519 Odenthal (DE)

(56) Entgegenhaltungen:
- DD-A- 288 595
- GB-A- 1 384 065

## Beschreibung

Die Erfindung betrifft ein neues Verfahren zur Reinigung von organischen Isocyanaten oder Isocyanatgemischen durch Vermischen der Isocyanate oder Isocyanatgemische mit Alkoholen und/oder Thiolen oder Gemischen von Alkoholen und/oder Thiolen und gegebenenfalls gleichzeitiger oder anschließender Entgasung und/oder destillativer und/oder extraktiver Aufarbeitung der so enthaltenden Gemische.

Herstellbedingte Verunreinigungen wechselnder Art und Menge in organischen Isocyanaten sind Anlaß für Aktivitätsschwankungen. Solche Aktivitätsschwankungen sind ungünstig für eine reproduzierbare, das heißt wirtschaftliche, Verwendung. So enthalten sowohl aromatische Isocyanate, zum Beispiel die bekannten Phosgenierungsprodukte von Anilin-Formaldehyd-Kondensaten oder auch 2,4- und 2,6-Disocyanatotoluol, aber auch aliphatische Isocyanate, wie zum Beispiel Isophorondiisocyanat, eine Fülle derartiger Verunreinigungen. Es handelt sich hierbei vor allem um Chlor-enthaltende Verbindungen, die immer dann Aktivitätsschwankungen Verursachen,wenn es sich um "leicht bewegliches", sogenanntes hydrolisierbares Chlor handelt Ein Teil dieser Verbindungen erweist sich als relativ stabil und verbleibt auch nach Destillation in den Isocyanaten. Er beeinflußt neben der Aktivität auch die Stabilität der Isocyanate ungünstig. Ein einheitlicher, niedriger Anteil dieser Verunreinigungen, mit der Folge einer Aktivitätsnormierung und leichteren Weiterverarbeitung, der Isocyanate ist daher sowohl von technischer wie auch von wirtschaftlicher Bedeutung.

Es hat daher nicht an Versuchen gefehlt, Möglichkeiten zur Entfernung der chlorhaltigen Verbindungen zu finden. So werden in den Patentschriften JP 4 501 032 9 B, JP 4 200 413 7 B, JP 5 908 845 2 A, JP 5 910 875 3 A, JP 5 917 245 0 A, US-A 3 373 182, GB-A 1 111 581, US-A 3 759 971, US-A 4 094 894, ZA 8 100 606, DE-A 11 38 040, DE-A 12 86 025, US-A 3 458 558, US-A 3 264 336, SU 8 066 77 und DE-A 22 10 607 auf Metallen oder Alkalimetallen basierte Zusätze beschrieben, wie z.B. Metalloxide, Metallcyanamide, Metallhydride, Metallfettsäureester in Gegenwart von sterisch gehinderten Phenolen, Metallnaphthenate, Metallsilikate, Alkalimetallcarbonate oder Organometallverbindungen. Alle genannten Verfahren führen zu einer Reihe technologischer Schwierigkeiten, die die Abtrennung der metallhaltigen Zusätze bzw. die eingeschränkte Verwendung metallhaltiger Isocyanate und/oder Destillationsrückstände betreffen.

Ähnliche Beschränkungen findet man bei der Verwendung von Zusätzen wie dem in GB-A 1 347 647 und JP 0 505 898 2 A beschriebenen Imidazol, den in GB-A 1 458 747 beschriebenen Sulfonsäuren und deren Estern, den in GB 1 459 691 beschriebenen Diethylsulfat und der auch dort beschriebenen Schwefelsäure, dem in DD 288 596 beschriebenen Trialkylphosphat und der Verwendung von anderen Zusätzen wie zum Beispiel Epoxyverbindungen (DE-A 22 49 375; JP 0 932 396 8 A2), tetrasubstituierten Harnstoffen (DD 288 598), Ameisen- oder Essigsäure oder deren Derivaten (US-A 3 799 963) oder den in der EP-A 524 507 beschriebenen Trimethylsilylgruppen aufweisenden Verbindungen.

Einige Verbindungen mit mindestens einer Zerevitinov-aktiven NH-Gruppe, wie Harnstoffe (DD 285 594), Biurete (DD 288 597), Caprolactam (DD 285 593), sowie Ammoniumsalze (DD 288 594), Carbodiimide (DD 288 599), primäre und sekundäre Aminsalze (DD 288 593), tertiäre Alkohole und tertiäre Alkylcarbamate (DD 288 595), werden nach dem Stand der Technik zur Reinigung von Isocyanaten empfohlen. Auch hier ist die Abtrennung der Zusätze bzw. die eingeschränkte Verwendung der zusatzhaltigen Isocyanate und/oder Destillationsrückstände, sowie besonders die teilweise starke Absenkung des NCO-Wertes und die Steigerung in der Viskosität, die sich zum Beispiel auf die Bildung von Biureten bei der Verwendung von tertiären Alkoholen zurückführen läßt, nachteilig. Letzteres gilt insbesondere auch für die Verwendung von Wasser zur Reinigung von Isocyanaten (DE-A 12 40 849).

Aus den Publikationen JP 6 116 125 0 A, JP 0 516 323 1 A, DE-A 19 50 101, DE-A 19 38 384, DE-A 25 32 722, DE-A 26 31 168, US-A 853 936, FR-A 1 555 517, DE-A 29 33 601, US-A 3 549 504 ist bekannt, daß Isocyanate durch spezielle Destillations- und Kristallisationstechniken gereinigt werden können.

Weiterhin ist bekannt, daß Erhitzen von Isocyanaten, insbesondere bei gleichzeitigem Strippen mit Inertgas, oder Erhitzen in inerten Lösungsmitteln unter Druck, bei gleichzeitigem Absaugen der flüchtigen Verbindungen, den Gehalt an leichtspaltbaren Chlorverbindungen vermindert (z. B. DE-A 12 70 036, DD 271 820, US-A 3 219 678, GB-A 1 080 717, DE-A 22 37 552; US-A 3 857 871, USA 1 458 223, JP 0 727 808 8 A2, JP 0 634 570 7 A2, GB 1 384 065), wobei allerdings vorwiegend der Gehalt an leicht spaltbaren Chlorverbindungen vermindert wird, der analytisch als Acidität erfaßt wird, während kurzzeitiges Erwärmen die Sedimentbildung unterdrücken kann (US-A 3 274 225).

Es war daher Aufgabe der vorliegenden Erfindung, ein neues Verfahren zur Reinigung von organischen Isocyanaten zur Verfügung zu stellen, welches die angesprochenen Mängel nicht aufweist. Diese Aufgabe konnte mit dem nachstehend näher beschriebenen erfindungsgemäßen Verfahren gelöst werden. Das Prinzip des erfindungsgemäßen Verfahrens besteht darin, den zu behandelnden organischen Isocyanten eine geringe Menge von nachfolgend näher beschriebenen Verbindungen gemäß Formel (I) zuzusetzten, das Gemisch für eine bestimmte Zeit, bei Bedarf unter erhöhtem oder vermindertem Druck, zu erhitzen, gleichzeitig oder anschließend mit einem Inertgas zu strippen und/oder durch eine einfache Destillation und/oder Extraktion aufzureinigen.

Gegenstand der vorliegenden Erfindung ist ein neues Verfahren zur Reinigung von organischen Isocyanaten oder Isocyanatgemischen, dadurch gekennzeichnet, daß man organische Isocyanate oder Isocyanatgemische mit insgesamt 0,01 bis 10 Gew.-%, bezogen auf die Menge der organischen Isocyanate oder Isocyanatgemische, an Verbindungen der Struktur gemäß Formel (I) oder Gemischen von Verbindungen der Struktur gemäß Formel (I) 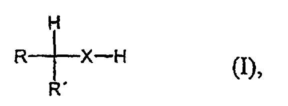 in welcher
- X: für O, S und
- R, R': unabhängig voneinander für H, C₁-C₃₆-Alkyl, C₆-C₃₆-Aryl, C₃-C₃₆-Cycloalkyl und C₇-C₃₆-Aralkyl
stehen,
wobei die Reste R und R' sowohl über einen Cyclus verbunden sein können als auch in den Resten R und/oder R' sowohl weiteren primäre und sekundäre X-H Gruppen als auch weitere nicht mit Isocyanaten reagierende funktionelle Gruppen sein können,
oder Phenolen,
für mindestens 5 min auf 100 bis 250°C mit oder ohne einem inerten, gegenüber Isocyanatgruppen nicht reaktiven Lösungsmittel, bei Bedarf unter erhöhtem oder vermindertem Druck, erhitzt, gleichzeitig oder anschließend mit einem Inertgas strippt und/oder destillativ und/oder extraktiv aufarbeitet.

Die erfindungsgemäß gereinigten, organischen Isocyanate eignen sich als Ausgangsmaterial bei der Herstellung von Polyurethankunststoffen nach den Isocyanat-Polyadditionsverfahren, insbesondere bei der Herstellung von Polyisocyanaten, die als Härter bei der Herstellung von Überzügen Verwendung finden.

Ausgangsmaterialien für das erfindungsgemäße Verfahren sind beliebige organische Isocyanate, wie
a) Monoisocyanate mit aliphatisch, cycloaliphatisch, araliphatisch oder aromatisch gebundenen Isocyanatgruppen (z. B. Butylisocyanat, Cyclohexylisocyanat, Phenylisocyanat) oder beliebige Gemische solcher Monoisocyanate;
b) Diisocyanate oder höherfunktionelle Isocyanate des Molekulargewichtbereichs 140 bis 400 mit aliphatisch, cycloaliphatisch, araliphatisch und/oder aromatisch gebundenen Isocyanatgruppen, wie z. B. 1,4-Diisocyanatobutan, 1,6-Diisocyanatohexan (HDI), 2-Methyl-1,5-diisocyanatopentan, 1,5-Diisocyanato-2,2-dimethylpentan, 2,2,4- bzw. 2,4,4-Trimethyl-1,6-diisocyanatohexan, 1,10-Diisocyanatodecan, 1,3- und 1,4-Diisocyanatocyclohexan, 1,3-und 1,4-Bis-(isocyanatomethyl)-cyclohexan, 1-Isocyanato-3,3,5-trimethyl-5-isocyanatomethylcyclohexan (Isophorondiisocyanat, IPDI), 4,4'-Diisocyanatodicyclohexylmethan, 1-Isocyanato-1-methyl-4(3)isocyanatomethyleyclohexan (IMCI), Bis-(isocyanatomethyl)-norbornan, 2-Methylpentan-2,4-diisocyanat, 1,3- und 1,4-Bis-(2-isocyanatoprop-2-yl)-benzol (TMXDI), 2,4- und 2,6-Diisocyanatotoluol (TDI), 2,4'- und 4,4'-Diisocyanatodiphenylmethan, 1,5-Diisocyanatonaphthalin, Dipropylenglycoldiisocyanat, 4-Isocyanatomethyl-1,8-octandiisocyanat (Nonantriisocyanat) oder beliebige Gemische solcher Diisocyanate und/oder höherfunktioneller Isocyanate.

Bevorzugt kommen bei dem genannten Verfahren die genannten di- und höherfunktionellen Isocyanate zum Einsatz. Ganz besonders bevorzugt die Diisocyanate mit aliphatisch und cycloaliphatisch gebundenen Isocyanatgruppen.

Bei den für das erfindungsgemäße Verfahren verwendeten Verbindungen handelt es sich um Verbindungen gemäß Formel (I) oder Mischungen aus Verbindungen gemäß Formel (I). Dazu gehören ein- oder mehrwertige gesättigte oder ungesättigte, cyclische, sowie lineare und verzweigte, primäre und sekundäre Alkohole, die bevorzugt 1 bis 36, besonders bevorzugt 1 bis 10 Kohlenstoffatome enthalten. Beispiele von solchen Monoalkoholen beinhalten: Methanol, Ethanol, n-Propanol, Isopropanol, n-Butanol, Isobutanol, n-Pentanol, 2-Hydroxypentan, 3-Hydroxypentan, die Isomeren primären und sekundären Methylbutylalkohole, n-Hexanol, n-Heptanol, n-Octanol, n-Nonanol, 2-Ethylhexanol, primäre und sekundäre Trimethylhexanole, Cyclohexanol, Benzylalkohol, n-Decanol, n-Undecanol, n-Dodecanol (Laurylalkohol), n-Tetradecanol, n-Pentadecanol, n-Hexadecanol, n-Heptadecanol, n-Octadecanol (Stearylalkohol), 2,6,8-Trimethylnonanol, 4-Cyclohexyl-1-butanol, 2,4,6-Trimethylbenzylalkohol, verzweigte und lineare, primäre und sekundäre Alkohole und Mischungen dieser, wie sie zum Beispiel von der Fa. Henkel unter dem Handelsnamen Lorol vertrieben werden, Glycerin oder andere zwei- oder höherwertige Alkohole mit primären und/oder sekundären OH-Gruppen.

Ebenso gehören zu den für das erfindungsgemäße Verfahren verwendeten Verbindungen gemäß Formel (I) ein- oder mehrwertige gesättigte oder ungesättigte, lineare und verzweigte primäre und sekundäre Thioalkohole die ebenfalls bevorzugt 1 bis 36, besonders bevorzugt 1 bis 10 Kohlenstoffatome enthalten und auch als Mischungen eingesetzt werden können.

Die verwendeten Verbindungen der Struktur gemäß Formel (I) können, wenn auch weniger bevorzugt, neben der XH-Gruppe (X=O,S) auch noch weitere funktionelle Gruppen, die nicht gegenüber der Isocyanatgruppe reaktiv sind, wie Ester, Ether oder Heteroatome wie Halogene (Cl, Br) oder Stickstoff, enthalten.

Selbstverständlich können neben Mischungen von Alkoholen gemäß Formel (I) und Mischungen von Thioalkoholen gemäß Formel (I), auch Mischungen von Alkoholen und Thioalkoholen gemäß Formel (I) im erfindungsgemäßen Verfahren verwendet werden, und natürlich können, wenn auch weniger bevorzugt, auch Lösungen von Verbindungen gemäß Formel (I) oder Lösungen von Mischungen von Verbindungen gemäß Formel (I) in inerten, das heißt nicht gegenüber der Isocyanatgruppe reaktiven Lösungsmitteln, eingesetzt werden.

Bevorzugt werden jedoch aliphatische, primäre C₁-C₁₀-Monoalkohole oder Mischungen von aliphatischen primären C₁-C₁₀-Monoalkoholen verwendet. Ganz besonders bevorzugt ist die Verwendung von n-Butanol.

Das Verfahren ist auch mit Phenolen durchführbar.

Das vorliegende erfindungsgemäße Verfahren sieht vor, das Gemisch aus organischem Isocyanat und der Verbindung gemäß Formel (I), gegebenenfalls aus dem Isocyanatgemisch und einer Verbindung gemäß Formel (I), dem Isocyanat und der Mischung von Verbindungen gemäß Formel (I) oder den Isocyanatgemisch und der Mischung von Verbindungen gemäß Formel (I), mit einem Anteil der Mischung von Verbindungen gemäß Formel (I) bzw. einer Verbindung gemäß Formel (I) von 0,01 bis 10 Gew.-%, bevorzugt 0,05 bis 5 Gew.-% , besonders bevorzugt 0,1 bis 2,5 Gew.-%, bezogen auf das Isocyanat bzw. das Isocyanatgemisch, ohne oder mit einem inerten, gegenüber Isocyanatgruppen nicht reaktiven, Lösungsmittel über einen Zeitraum von mindestens 5 min, vorzugsweise mindestens 240 min, bei Bedarf unter erhöhtem oder vermindertem Druck, auf eine Temperatur von 100 bis 250°C, vorzugsweise mindestens 150°C, besonders bevorzugt über 180°C, zu erhitzen, während des Erhitzens oder danach mit einem Inertgas zu strippen und/oder destillativ und/oder extraktiv aufzuarbeiten. Unter der destillativen Aufarbeitung ist im Falle der Verwendung von destillierbaren Ausgangsisocyanaten deren destillative Reindarstellung, beispielsweise im Dünnschichtverdampfer oder über eine Destillationsbrücke, zu verstehen.

Anstelle des Strippens mit Inertgas kann selbstverständlich genauso bevorzugt auch eine Entgasung im Vakuum (beispielsweise 100 bis 1 mbar) durchgeführt werden.

Die für die im erfindungsgemäßen Verfahren eingesetzten Verbindungen gemäß Formel (I) oder deren Mischungen benannten Gewichtsprozente können in Abhängigkeit vom Äquivalentgewicht der Verbindungen gemäß Formel (I) varieren.

Ein NCO/XH-Verhältnis [mit X = O,S] von 10 zu 1 wird im erfindungsgemäßen Verfahren nicht unterschritten.

Die erfindungsgemäß behandelten organischen Isocyanate weisen verringerte Aktivitätsschwankungen auf. Dies läßt sich besonders einfach anhand verringerter Werte an hydrolysierbarem Chlor, gleichbedeutend mit zum Beispiel verbesserten Aktivitäten bei vergleichender Verschäumung, oder verbesserter Farbstabilität nachweisen.

### Beispiele

Die angegebenen HC-Werte beziehen sich auf den Gehalt an hydrolisierbarem Chlor. Alle Prozentangaben beziehen sich auf das Gewicht.

### Beispiele

100 g des organischen Isocyanates werden mit der angegebenen Menge der in Tabelle 1 bis 3 angegebenen Verbindung versetzt, und unter schwachem Inertgasstrom wird die angegebene Zeitspanne bei der angegebenen Temperatur gerührt (=Konditionierung). Nach dem Abkühlen erhält man die erfindungsgemäßen Produkte, die optional zur weiteren Aufreinigung destilliert werden können.

Das im erfindungsgemäßen Beispiel 6 (Tabelle 2) erhaltene Produkt wird über eine Destillationsbrücke destilliert (Kopftemperatur 96°C; Druck: 1,5*10-2 mbar), und man erhält 99,8 %iges (GC), farbhelles Isophorondiisocyanat.

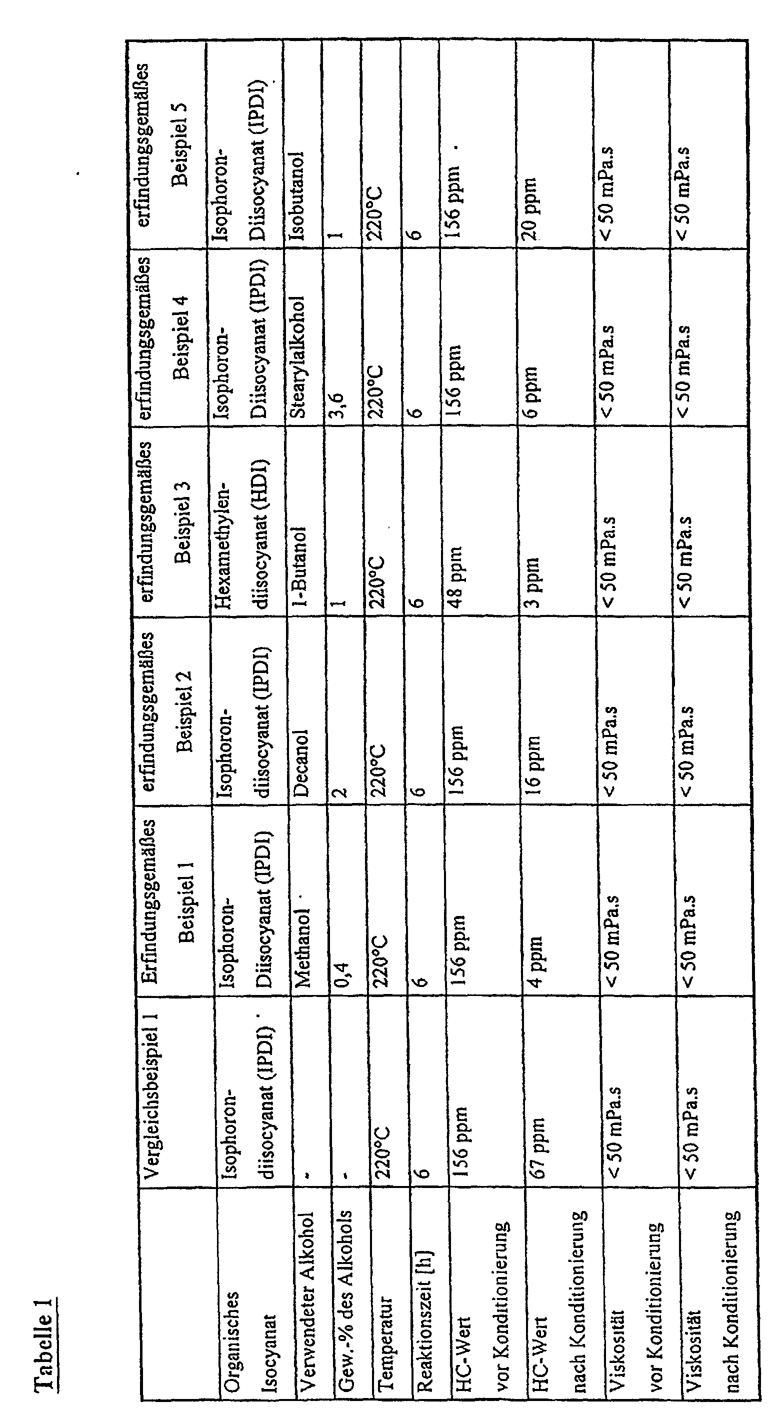

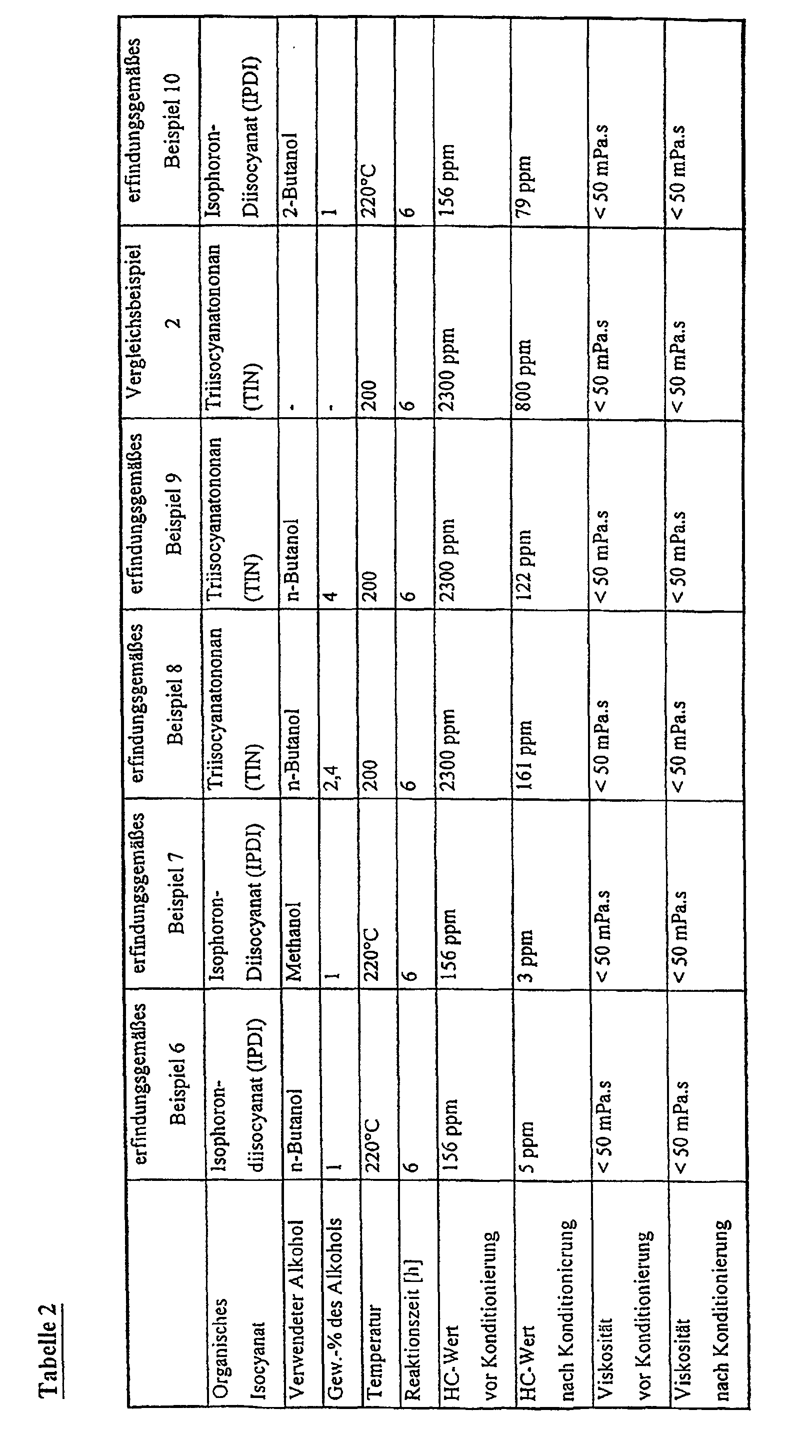

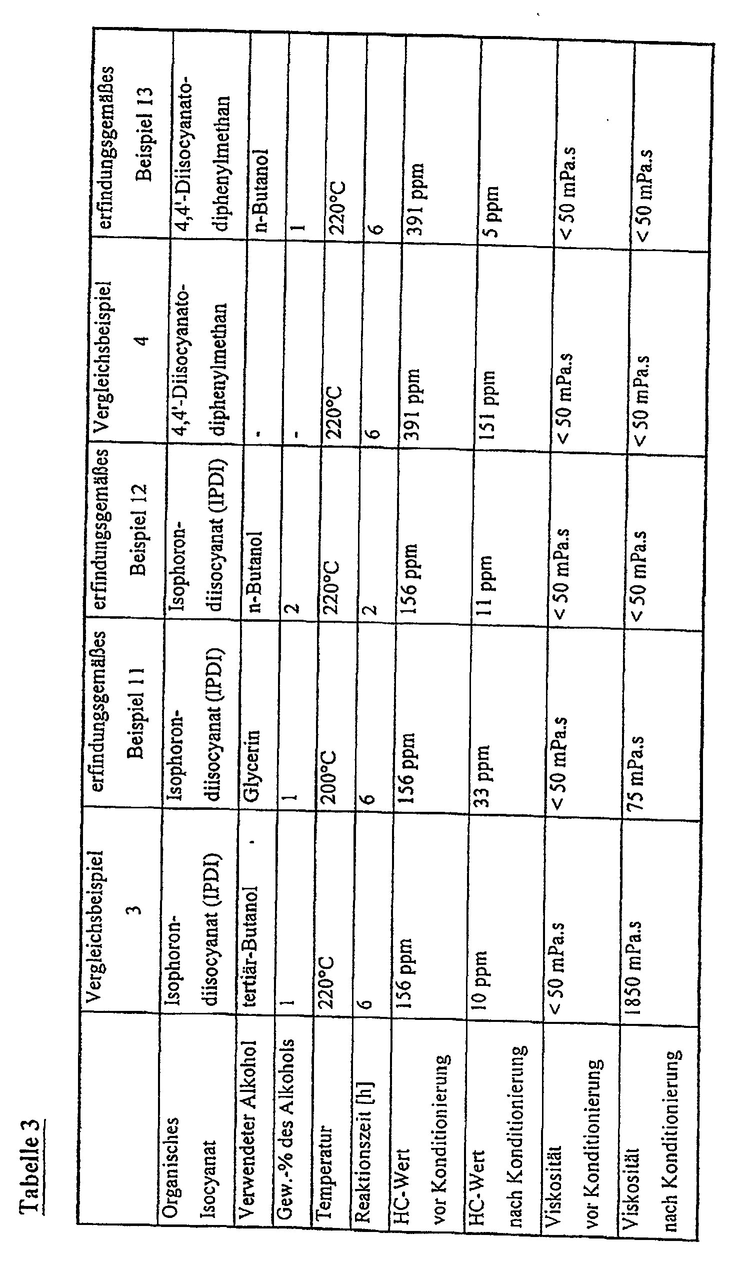

Die Vergleichsbeispiele 1,2 und 4 zeigen, daß ohne den erfindungsgemäßen Zusatz einer Verbindung gemäß Formel (I) eine nicht ausreichende Reinigung der Isocyanate gelingt, was an relativ hohem HC-Wert nach der Konditionierung festzustellen ist. Dagegen werden durch Zugeben schon einer geringen Menge einer Verbindung gemäß Formel (I) (erfindungsgemäßes Beispiel 1) sehr niedrige HC-Werte erreicht.

Vergleichsbeispiel 3 zeigt einen untragbaren Anstieg der Viskosität bei der Verwendung von tertiär-Butanol gemäß DD 288 595. Bei der erfindungsgemäßen Verwendung einer Verbindung gemäß Formel (I) bleibt die Viskosität wesentlich niedriger (Beispiel 12).

## Patentansprüche

1. Verfahren zur Reinigung von organischen Isocyanaten oder Isocyanatgemischen, **dadurch gekennzeichnet, daß** man organische Isocyanate oder Isocyanatgemische mit insgesamt 0,01 bis 10 Gew.-%, bezogen auf die Menge der organischen Isocyanate oder Isocyanatgemische, an Verbindungen der Struktur gemäß Formel (I) oder Gemischen von Verbindungen der Struktur gemäß Formel (I) 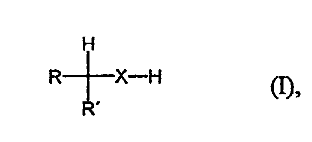 in welcher
X für O, S und
R, R' unabhängig voneinander für H, C₁-C₃₆-Alkyl, C₆-C₃₆-Aryl, C₃-C₃₆Cycloalkyl und C₇-C₃₆-Aralkyl
stehen,
wobei die Reste R und R' sowohl über einen Cyclus verbunden sein können als auch in den Resten R und/oder R' sowohl weiteren primäre und sekundäre X-H Gruppen als auch weitere nicht mit Isocyanaten reagierende funktionelle Gruppen sein können,
oder Phenolen,
für mindestens 5 min auf 100 bis 250°C mit oder ohne einem inerten, gegenüber Isocyanatgruppen nicht reaktiven Lösungsmittel, bei Bedarf unter erhöhtem oder vermindertem Druck, erhitzt, gleichzeitig oder anschließend mit einem Inertgas strippt und/oder destillativ und/oder extraktiv aufar beitet.

2. Verfahren nach Anspruch 1, **dadurch gekennzeichnet, daß** als Verbindung der Formel (I) primäre Alkohole eingesetzt werden.

3. Verfahren nach Anspruch 1, **dadurch gekennzeichnet, daß** n-Butanol als Alkohol eingesetzt wird.

## Claims

1. A process for purifying organic isocyanates or isocyanate mixtures, **characterised in that** organic isocyanates or isocyanate mixtures are heated with a total of 0.01 to 10 wt.%, with respect to the amount of organic isocyanate or isocyanate mixture, of compounds with a structure in accordance with formula (I) or mixtures of compounds with structures in accordance with formula (I) 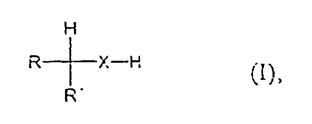 in which
X represents O, S and
R, R', independently, represent H, or a C₁-C₃₆-alkyl, C₆-C₃₆-aryl, C₃-C₃₆-cycloalkyl or C₇-C₃₆-aralkyl group,
wherein the groups R and R' may be linked via a ring system and also either further primary and secondary X-H groups or other functional groups which do not react with isocyanates may be present in groups R and/or R',
or phenols,
for at least 5 min at 100 to 250°C with or without an inert solvent which does not react with isocyanate groups, if required under elevated or reduced pressure, and at the same time or subsequently is stripped with an inert gas and/or is worked up by distillation and/or extraction.

2. A process according to Claim 1, **characterised in that** primary alcohols are used as compounds of the formula (I).

3. A process according to Claim 1, **characterised in that** n-butanol is used as an alcohol.

## Revendications

1. Procédé pour la purification d'isocyanates
ou de mélanges d'isocyanates organiques, **caractérisé en ce que** l'on chauffe les isocyanates ou mélanges d'isocyanates organiques avec au total, 0,01 à 10% en poids, sur base de la quantité d'isocyanate ou de mélange d'isocyanates organique, de composés de la structure de formule (I) ou de mélanges de composés de structure de formule (I) : 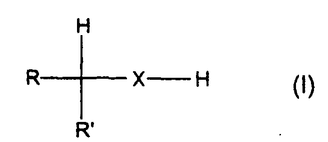 dans laquelle :
X représente O, S et
R, R' représentent indépendamment l'un de l'autre, H, un radical alcoyle en C₁-C₃₆, aryle en C₆-C₃₆, cycloalcoyle en C₃-C₃₆, et aralcoyle en C₇-C₃₆,
où les restes R et R' peuvent être reliés par un cycle, mais également dans les restes R et/ou R', il peut y avoir d'autres radicaux XH primaires et secondaires et également, d'autres groupes fonctionnels ne réagissant pas avec les isocyanates,
ou des phénols,
pendant au moins 5 minutes à 100°C à 250°C avec ou sans solvant inerte, non réactif avec les radicaux isocyanate, selon le besoin sous pression augmentée ou réduite, simultanément ou ensuite on lave avec un gaz inerte et/ou traite par distillation et/ou par extraction.

2. Procédé suivant la revendication 1, **caractérisé en ce que** comme composé de la formule (I), on met en oeuvré un alcool primaire.

3. Procédé suivant la revendication 1, **caractérisé en ce que** l'on met en oeuvre le n-butanol comme alcool.
